# EUROPEAN PATENT SPECIFICATION

(11) **EP 0 715 610 B1**
(45) Date of publication and mention of the grant of the patent: **20.10.1999**
(21) Application number: 94924543.5
(22) Date of filing: 01.08.1994
(51) Int. Cl.: C07C 2/56, C07C 2/58, C07C 2/62, C07C 7/00, C07C 7/10

(54) **ISOPARAFFIN-OLEFIN ALKYLATION**
ISOPARRAFIN-OLEFIN-ALKYLIERUNG
ALKYLATION D'ISOPARAFFINE/OLEFINE

(30) Priority: 23.08.1993 US 111221; 07.09.1993 US 117274
(43) Date of publication of application: 12.06.1996
(73) Proprietor: PHILLIPS PETROLEUM COMPANY, Bartlesville Oklahoma (US)
(72) Inventor: CHILD, Jonathan, Edward, Sewell, NJ 08080-1660 (US); MELLI, Tomas, Rodolfo, Sewell, NJ 08080-3311 (US); YURCHAK, Sergei, Upper Providence Township, PA 19063-1133 (US)
(74) Representative: Dost, Wolfgang, Dr.rer.nat., Dipl.-Chem.
(86) International application number: US9408688
(87) International publication number: WO9506017

(56) References cited:
- US-A- 5 191 150
- US-A- 5 237 122
- US-A- 5 262 579
- US-A- 5 264 647
- US-A- 5 264 649
- US-A- 5 264 650
- US-A- 5 264 651
- US-A- 5 264 652
- US-A- 5 276 243

## Description

The present invention relates to an isoparaffin-olefin alkylation process. Alkylation is a reaction in which an alkyl group is added to an organic molecule. Thus an isoparaffin can be reacted with an olefin to provide an isoparaffin of higher molecular weight. Industrially, the concept depends on the reaction of a C₂ to C₅ olefin with isobutane in the presence of an acidic catalyst producing a so-called alkylate. This alkylate is a valuable blending component in the manufacture of gasolines due not only to its high octane rating but also to its sensitivity to octane-enhancing additives.

Industrial alkylation processes have historically used concentrated hydrofluoric or sulfuric acid catalysts under relatively low temperature conditions. Acid strength is preferably maintained at 88 to 94 weight percent by the continuous addition of fresh acid and the continuous withdrawal of spent acid. As used herein, the term "concentrated hydrofluoric acid" refers to an essentially anhydrous liquid containing at least 85 weight percent HF.

Hydrofluoric and sulfuric acid alkylation processes share inherent drawbacks including environmental and safety concerns, acid consumption, and sludge disposal. For a general discussion of sulfuric acid alkylation, see the series of three articles by L.F. Albright et al., "Alkylation of Isobutane with C₄ Olefins", 27 Ind. Eng. Chem. Res., 381-397, (1988). For a survey of hydrofluoric acid catalyzed alkylation, see 1 Handbook of Petroleum Refining Processes 23-28 (R.A. Meyers, ed., 1986).

Hydrogen fluoride, or hydrofluoric acid (HF) is highly toxic and corrosive. However, years of experience in its manufacture and use have shown that HF can be handled safely, provided the hazards are recognized and precautions taken. Though many safety precautions are taken to prevent leaks, massive or catastrophic leaks are feared primarily because the anhydrous acid will fume on escape creating a vapor cloud that can be spread for some distance.

Previous workers in this field have approached this problem from the standpoint of containing or neutralizing the HF cloud after its release. Thus U.S. Patents 4,938,935 and 4,985,220 to Audeh and Greco, as well as U.S. Patent 4,938,936 to Yan teach various methods for containing and/or neutralizing HF acid clouds following accidental releases. In addition, it has been proposed to provide an additive which decreases the cloud forming tendency of HF without compromising its activity as an isoparaffin-olefin alkylation catalyst. For example, WO93/00314 discloses a method for increasing the rainout from an autorefridgerated vaporour cloud containing HF by admixing the HF with a sulfone component, preferably sulfolane.

Isoparaffin-olefin alkylation processes typically convert at least a portion of the feedstock to conjunct polymeric byproducts, which are more commonly referred to as acid soluble oil or ASO. Adding sulfolane to HF for isoparaffin-olefin alkylation complicates the problem of removing ASO from the system because the typical boiling range of the ASO brackets the boiling point of sulfolane (285°C, 545°F). Thus sulfolane cannot be readily separated from ASO by distillation.

U.S. Patent 5,191,150 teaches a sulfolane recovery method which involves reducing the HF concentration in a mixture of HF, sulfolane, and ASO to less than about 30 weight percent and then gravitationally separating the resulting mixture to recover sulfolane. The HF-enriched stream evolved from the stripping step of this process contains a minor amount of relatively low boiling range ASO byproducts which are recycled to the alkylation reaction zone.

Minimizing ASO concentration in the alkylation reaction zone has been found to improve isoparaffin-olefin alkylation in the presence of HF and sulfolane. Thus it would be desirable to improve the process of U.S. Patent 5,191,150 by decreasing the concentration of ASO in the HF-enriched stream recycled to the alkylation reaction zone.

The present invention provides a method separating a mixture of HF, sulfolane, and conjunct polymeric byproducts formed in HF/sulfolane-catalyzed isoparaffin-olefin alkylation, which method decreases the concentration of conjunct polymeric byproducts (ASO) recycled to the alkylation reaction zone with the recycled HF.

Accordingly, the present invention resides in an isoparaffin-olefin alkylation process comprising the sequential steps of:
(a) alkylating an isoparaffin with an olefin in the presence of an alkylation catalyst comprising HF and sulfolane in an alkylation reaction zone whereby ASO byproduct is evolved;
(b) gravitationally separating effluent from said alkylation reaction zone to provide a less-dense stream containing alkylate product and unreacted isoparaffin and a more dense stream containing sulfolane, ASO, and HF;
(c) stripping HF from said more dense stream of step (b) with a stripping fluid in a multistage stripper column to provide a stripper bottoms stream containing less than 30 percent HF by weight and a stripper overhead stream containing HF, isoparaffin, and a fraction of said ASO having a lower end boiling point than the ASO contained in said more dense stream of step (b);
(d) gravitationally separating said stripper bottoms stream into a more dense sulfolane-enriched stream and a less dense ASO-enriched stream;
(e) charging said stripper overhead stream to an alkylate product fractionator;
(f) recovering an overhead stream containing isoparaffin and HF from said alkylate product fractionator;
(g) recycling said overhead stream of step (f) to said alkylation reaction zone; and
(h) recovering from said alkylate product fractionator an alkylate product stream containing alkylate gasoline and a fraction of said ASO having a lower end boiling point than the ASO contained in said more dense stream of step (b).

In the process of the invention, the hydrofluoric acid concentration of the more dense product fraction is decreased by stripping. Any suitable inert stripping fluid may be employed, including normal paraffins and isoparaffins which can be charged to the stripper tower as a vapor. Isobutane, normal butane and the vaporized alkylate product formed by reacting isobutane with propene and/or butene are particularly preferred stripping fluids. Two sequential stripping steps may be used, as the purity of the separated sulfolane/conjunct polymer phases improves as the hydrofluoric acid concentration decreases. If two-stage stripping is used, the enriched stripping fluid from both stripping stages is preferably charged to the product fractionator.

The surprising effects of sequentially stripping hydrofluoric acid from the mixture before gravitational separation become particularly evident as the mixture is stripped to hydrofluoric acid levels of less than 30 weight percent. Separation improves as the hydrofluoric acid content is decreased, with intermediate stream hydrofluoric acid concentrations preferably falling below 25 percent by weight, more preferably below 10 percent hydrofluoric acid by weight, and most preferably below 5 percent by weight. In a preferred embodiment, the catalyst mixture contains from 0.5 to 10 weight percent water.

The ASO byproducts of liquid acid catalyzed isoparaffin-olefin alkylation are understood to comprise a complex mixture, but the mechanism underlying the present invention is not well understood. The stripping step of the present invention splits the ASO between two substantially immiscible liquid phases. The ASO fraction in the more-dense phase boils generally above the normal boiling point of sulfolane, allowing the sulfolane to be recovered as the overhead stream from a distillation tower. The less-dense phase, on the other hand is enriched in ASO and may be treated further to remove sulfolane and/or for disposal. The fraction of ASO which is stripped out of the HF/sulfolane/ASO mixture in the catalyst stripper tower boils at a lower ranges of temperatures than the total ASO fraction flowing to the catalyst stripper. Accumulating this light ASO fraction is, nontheless, detrimental to catalyst performance in the alkylation riser/reactor and the present invention improves overall process performance by continuously removing light ASO from the system.

The invention will now be more particularly described with reference to the accompanying drawings, in which:

Figure 1 is a simplified schematic diagram showing initial processing steps in the method of the invention.

Figure 2A shows the infrared (IR) spectrum of the ASO from the lower-density phase withdrawn from the gravitation separation step of a process according to one example of the invention.

Figure 2B shows the IR spectrum of the higher density phase withdrawn from the gravitational separation step of a process according to said one example of the invention.

Figure 2C shows the IR spectrum of sulfolane extracted from the higher density phase withdrawn from the gravitational step of a process according to said one example of the invention.

Figure 3 shows a simulated distillation comparing the boiling ranges of components in the ASO from the lower density phase of the gravitational separation step with the ASO from the higher density phase of the gravitational separation step of a process according to said one example of the invention.

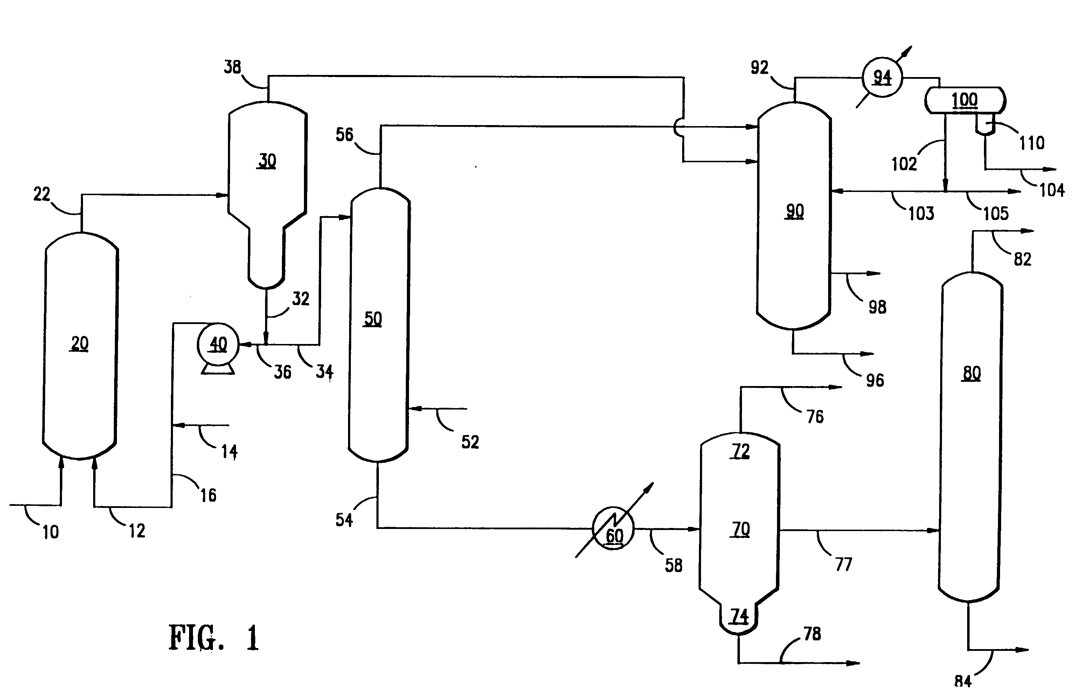

Referring now to Figure 1, mixed isoparaffin and olefin feed 10 and liquid catalyst 12 flow to riser/reactor 20. The riser/reactor effluent 22 flows to gravitational separator 30 where the effluent separates into a less dense hydrocarbon stream 38 containing alkylate and unreacted isoparaffin and a more dense catalyst stream 32 which contains HF, sulfolane, and ASO. The majority of the catalyst stream 32 recycles to riser/reactor 20 via stream 36, catalyst recycle pump 40, and stream 16. Fresh makeup HF and sulfolane enter stream 16 as required via stream 14. A minor amount of catalyst stream 32 flows to catalyst stripper 50 via stream 34. Isoparaffin (typically isobutane) from stream 52 strips HF and a lighter boiling fraction of the ASO from the catalyst mixture to produce a stripped catalyst stream 54 containing less than about 30 weight percent HF. The stripping fluid (isobutane), now enriched in HF and a lighter boiling fraction of the ASO, flows to product fractionator 90 as stream 56.

The stripped catalyst, stream 54, flows to cooler 60 from the catalyst stripper at tower temperature of about 150°C (300°F), and is cooled to about 20°C (70°F). The cooled stripped catalyst stream 58 enters gravitational separator 70 at approximately atmospheric pressure.

Two liquid phases form within gravitational separator 70. The upper, less dense phase, enriched in ASO, collects near the top 72 of gravitational separator 70, and is withdrawn through line 76 for further processing, as described below. Solids and the most dense residual hydrocarbons collect in a bottom boot 74, and are similarly withdrawn for further processing as stream 78. The lower, more dense liquid phase, enriched in sulfolane, flows out of gravitational separator 70 as stream 77 and enters a lower middle section of vacuum distillation column 80, which operates at a feed tray temperature of about 150°C (300°F) and the maximum available vacuum. The sulfolane and ASO readily separate in vacuum distillation column 80, with the sulfolane flowing overhead as stream 82 for recycle to riser/reactor 20 and the ASO leaving the column as stream 84.

Streams 38 and 56 flow to product fractionator 90, with stream 56, the isobutane stripping fluid enriched in HF and a lighter boiling fraction of the ASO, preferably entering product fractionator 90 on a tray above the feed tray for stream 38. The overhead stream 92 from product fractionator 90, enriched in isobutane and HF, condenses in overhead cooler 94 and separates into a hydrocarbon phase and an acid phase in overhead accumulator 100. The hydrocarbon phase, enriched in isobutane, leaves accumulator 100 as stream 102, and splits between reflux stream 103 and isobutane recycle stream 105. The acid phase in accumulator 100 settles in a lower boot section 110 of the accumulator and is withdrawn as stream 104 for recycle to riser/reactor 20. Alkylate product, containing a minor amount of light ASO, flows from product fractionator 90 as stream 96, while n-butane is withdrawn as side draw 98.

### Comparative Example

A mixture of hydrofluoric acid, sulfolane, and ASO (produced as the by-product of the catalytic alkylation of isobutane with butene) containing about 65 weight percent hydrofluoric acid, 30 weight percent sulfolane and about 5 weight percent ASO, is charged to a decantation vessel at ambient temperature and pressure sufficient to maintain the mixture in the liquid phase. The mixture is allowed to stand for approximately 24 hours. No phase separation is observed.

### Example 1

A mixture of hydrofluoric acid, sulfolane, and ASO (having the same composition as the mixture of the Comparative Example, above) is charged to a stripping tower having three theoretical stages. Isobutane is introduced into the tower at a level below the height of the liquid (HF/sulfolane/ASO) charge point, and the isobutane and mixture charge rates are controlled to maximize stripping of HF while operating below the flooding point of the tower. A stripped liquid is withdrawn from the bottom of the tower and a HF-enriched isobutane stream is withdrawn from the top of the tower. The stripped liquid contains less than 30 percent by weight of hydrofluoric acid.

The stripped liquid is then charged to a decantation vessel and allowed to stand for approximately 24 hours. The mixture separates into two distinct phases, an upper, less dense ASO-enriched phase, and a lower, more dense, sulfolane-enriched phase.

### Examples 2-4

Additional samples of the mixture of hydrofluoric acid, sulfolane, and ASO (having the same composition as the mixture of the Comparative Example) are stripped with isobutane to hydrofluoric acid contents of 25 weight percent, 10 weight percent, and 5 weight percent, respectively. The stripped mixtures containing lower concentrations of hydrofluoric acid separate more readily than mixtures having higher HF concentrations.

### Example 5

The HF/sulfolane sample of Example 5 has the following composition:

| | |
|---|---|
| HF | 62 wt. % |
| Sulfolane | 27 wt. % |
| Isobutane | 4 wt. % |
| Water | 1-2 wt. % |
| ASO | 3 wt. % |
| Balance to 100% other hydrocarbons. | |

This mixture is a single liquid phase at 32°C (90°F) and 930 kPa (120 psig).

The sample is brought to atmospheric pressure and room temperature and most of the light hydrocarbons and part of the HF are vented off. Under these conditions, the sample is a single liquid phase containing about 50 wt. % HF.

Nitrogen is then bubbled through the mixture at room temperature and atmospheric pressure to strip HF off the mixture. As the mixture is depleted in HF, the mixture separates into two phases.

Both phases are analyzed, and the dense phase (specific gravity about 1.26) contains 83.2 wt. % sulfolane, 2.2 wt. % ASO, and the balance water, salts, and a sludge. The lighter phase, having a density of less than about 1, contains 82.8 wt. % ASO, 13.3 wt. % sulfolane, and the balance of salts.

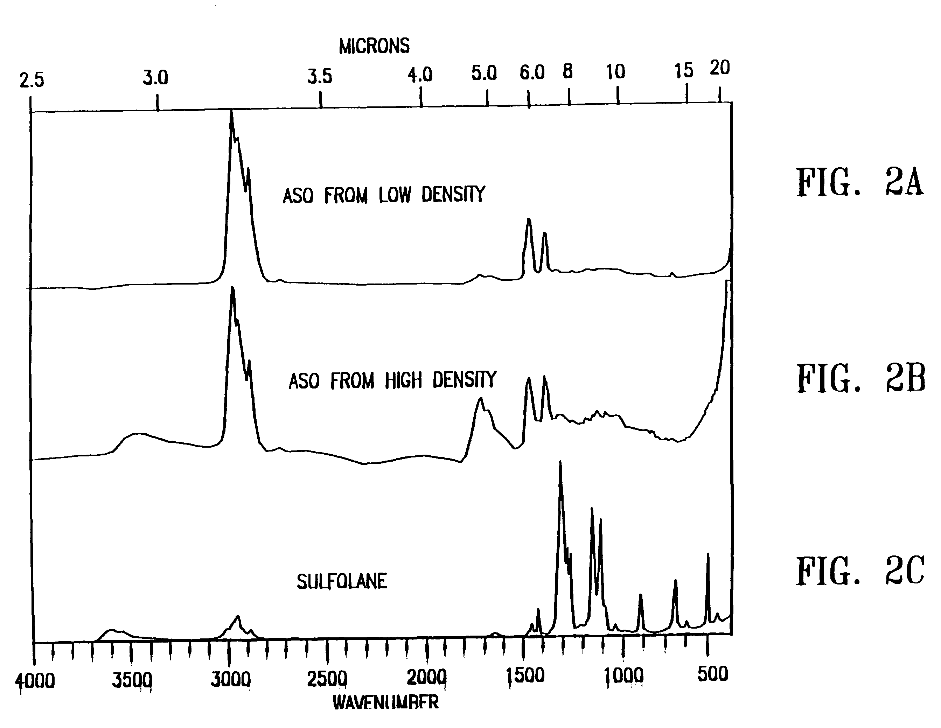

Figure 2 shows the IR spectra of ASO from the lighter phase (the upper spectrum), ASO from the heavier phase (the middle spectrum) and sulfolane (the lower spectrum).

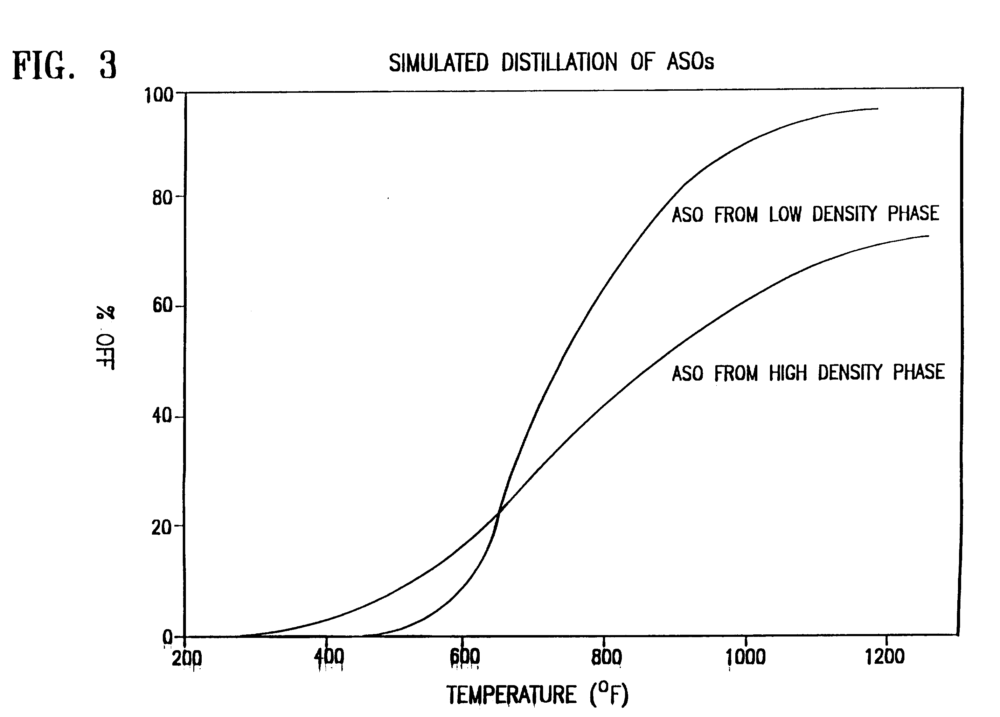

Figure 3 shows simulated distillations of ASO fractions from the low density phase and the high density phase from the gravitational separation step. The initial boiling point and the endpoint for the low density phase are both different from the corresponding points for the high density phase. Thus the gravitational separation splits the ASO into two fractions having different, albeit overlapping, boiling ranges.

### Example 6

The sulfolane-enriched dense phase of Example 5 is charged to a vacuum distillation column under the maximum available vacuum. The column bottom temperature is about 150°C (300°F). The overhead stream withdrawn from the distillation column is highly enriched in sulfolane while the bottoms product predominantly contains the higher boiling ASO fraction contained in the more-dense phase of Example 5.

### Example 7

A catalyst mixture containing about 65 wt.% HF, 30 wt.% sulfolane, and about 5 wt.% ASO is fed to a catalyst stripper column at a rate of about 320 m³/day (2,000 barrels per day, BPD). The catalyst stripper column operates at about 100 kPa (150 psi). Isobutane (as stripping fluid) is charged to the catalyst stripper tower at a rate of about 15890 kg/hr (35,000 lb/hr) to strip HF and a light fraction of the ASO from the catalyst mixture. The bottom stream from the catalyst stripper tower contains approximately 82 wt.% sulfolane and the balance HF, heavy ASO, and hydrocarbons. From the top of the catalyst stripper column, about 15890 kg/hr (35,000 lb/hr) of isobutane, 7718 kg/hr (17,000 lb/hr) of HF, and 363 kg/hr (800 lb/hr) of ASO at about 90°C (200°F) are sent to an upper (stripping) section of a main product fractionator.

The principal feeds to the main product fractionator are about 431,300 kg/hr (950,000 lb/hr) of hydrocarbon alkylation reactor effluent, which predominately comprises isobutane with about 15 wt.% alkylate. The overhead stream from the main product fractionator, about 340,500 kg (750,000 lb) of hydrocarbon and HF, is condensed and separated into two phases: an isobutane-rich phase saturated in HF and essentially free of ASO, and an HF phase, saturated in isobutane and essentially free of ASO.

A small side stream removes n-butane from the main product fractionator. The bottoms product, mainly alkylate and ASO, is sent to an alkylate product storage tank. Of the total charge to the product fractionator, the acid-rich feed from the top of the catalyst stripper column typically accounts for 3.5 to 4%, and the light ASO fraction typically comprises about 0.7 wt.% of the alkylate product stream withdrawn from the product fractionator.

## Claims

1. A isoparaffin-olefin alkylation process comprising the sequential steps of:
(a) alkylating an isoparaffin with an olefin in the presence of an alkylation catalyst comprising HF and sulfolane in an alkylation reaction zone whereby ASO byproduct is evolved;
(b) gravitationally separating effluent from said alkylation reaction zone to provide a less-dense stream containing alkylate product and unreacted isoparaffin and a more dense stream containing sulfolane, ASO, and HF;
(c) stripping HF from said more dense stream of step (b) with a stripping fluid in a multistage stripper column to provide a stripper bottoms stream containing less than 30 percent HF by weight and a stripper overhead stream containing HF, isoparaffin, and a fraction of said ASO having a lower end boiling point than the ASO contained in said more dense stream of step (b);
(d) gravitationally separating said stripper bottoms stream into a more dense sulfolane-enriched stream and a less dense ASO-enriched stream;
(e) charging said stripper overhead stream to an alkylate product fractionator;
(f) recovering an overhead stream containing isoparaffin and HF from said alkylate product fractionator;
(g) recycling said overhead stream of step (f) to said alkylation reaction zone; and
(h) recovering from said alkylate product fractionator an alkylate product stream containing alkylate gasoline and a fraction of said ASO having a lower end boiling point than the ASO containing in said more dense stream of step (b).

2. A process as claimed in claim 1 wherein said stripping fluid of step (c) comprises isobutane or normal butane.

3. A process as claimed in claim 1 wherein said stripping fluid of step (c) comprises an alkylated product formed by reacting an isoparaffin with an olefin.

4. A process as claimed in any preceding claim wherein said HF stripping step (c) provides an intermediate stream containing less than 25 percent HF by weight.

5. A process as claimed in any preceding claim wherein said HF stripping step (c) provides an intermediate stream containing less than 10 percent hydrofluoric acid by weight.

6. A process as claimed in any preceding claim wherein said HF stripping step (c) provides an intermediate stream containing less than 5 percent hydrofluoric acid by weight.

## Patentansprüche

1. Isoparaffin-Olefin-Alkylierungsverfahren, umfassend die folgenden sequenziellen Stufen:
(a) das Alkylieren eines Isoparaffins mit einem Olefin in Gegenwart eines Alkylierungskatalysators, der HF und Sulfolan umfaßt, in einer Alkylierungsreaktionszone, wobei ASO-Nebenprodukt entsteht;
(b) das Trennen unter Schwerkrafteinwirkung eines aus der Alkylierungsreaktionszone ausfließenden Stroms unter Bereitstellung eines Stroms von geringerer Dichte, der ein Alkylatprodukt und nicht-umgesetztes Isoparaffin enthält, und eines Stroms von höherer Dichte, der Sulfolan, ASO und HF enthält;
(c) das Abstreifen von HF aus dem Strom von höherer Dichte von Stufe (b) mit einem Abstreiffluid in einer mehrstufigen Abstreifkolonne unter Bereitstellung eines Abstreifbodenstroms, der weniger als 30 Gew.-% HF enthält und eines Abstreifüberkopfstroms, der HF, Isoparaffin und eine Fraktion des ASO, die im Vergleich zu dem ASO, das im Strom von höherer Dichte von Stufe (b) enthalten ist, einen niedrigeren Endsiedepunkt aufweist;
(d) das Trennen unter Schwerkrafteinwirkung des Abstreifbodenstroms in einen in bezug auf Sulfolan angereicherten Strom von höherer Dichte und einen in bezug auf ASO angereicherten Strom von geringerer Dichte;
(e) das Zuführen des Abstreifüberkopfstroms in eine Alkylatprodukt-Fraktioniervorrichtung;
(f) das Gewinnen eines Überkopfstroms mit einem Gehalt an Isoparaffin und HF aus der Alkylatprodukt-Fraktioniervorrichtung;
(g) das Rückführen des Überkopfstroms von Stufe (f) in die Alkylierungsreaktionszone; und
(h) das Gewinnen eines Alkylat-Produktstroms mit einem Gehalt an Alkylatbenzin und einer Fraktion des ASO, die im Vergleich zum im Strom von höherer Dichte von Stufe (b) enthaltenen ASO einen niedrigeren Endsiedepunkt aufweist, aus der Alkylatprodukt-Fraktioniervorrichtung.

2. Verfahren nach Anspruch 1, wobei das Abstreiffluid von Stufe (c) Isobutan oder n-Butan umfaßt.

3. Verfahren nach Anspruch 1, wobei das Abstreiffluid von Stufe (c) ein durch Umsetzung eines Isoparaffins mit einem Olefin gebildetes alkyliertes Produkt umfaßt.

4. Verfahren nach einem der vorstehenden Ansprüche, wobei die HF-Abstreifstufe (c) einen mittleren Strom mit einem Gehalt an weniger als 25 Gew.-% HF bereitstellt.

5. Verfahren nach einem der vorstehenden Ansprüche, wobei die HF-Abstreifstufe (c) einen mittleren Strom mit einem Gehalt an weniger als 10 Gew.-% Fluorwasserstoffsäure bereitstellt.

6. Verfahren nach einem der vorstehenden Ansprüche, wobei die HF-Abstreifstufe (c) einen mittleren Strom mit einem Gehalt an weniger als 5 Gew.-% Fluorwasserstoffsäure bereitstellt.

## Revendications

1. Procédé d'alkylation d'isoparaffine-oléfine comprenant les étapes séquentielles consistant à :
(a) alkyler une isoparaffine avec une oléfine en présence d'un catalyseur d'alkylation comprenant HF et du sulfolane dans une zone de réaction d'alkylation si bien que du sous-produit ASO est dégagé ;
(b) séparer par gravité l'effluent à partir de ladite zone de réaction d'alkylation pour obtenir un courant moins dense contenant l'alkylat et l'isoparaffine n'ayant pas réagi et un courant plus dense contenant du sulfolane, ASO et HF ;
(c) entraîner HF à partir dudit courant plus dense de l'étape (b) avec un fluide d'entraînement dans une colonne de dispositif d'entraînement à plusieurs étages pour avoir un courant de queue du dispositif d'entraînement renfermant moins de 30 % en poids de HF et un courant aérien de dispositif d'entraînement renfermant HF, de l'isoparaffine et une fraction de ladite ASO ayant un point d'ébullition final inférieur à celui de ASO contenue dans ledit courant plus dense de l'étape (b) ;
(d) séparer par gravité ledit courant de queue de dispositif d'entraînement selon un courant enrichi de sulfolane plus dense et un courant enrichi de ASO moins dense ;
(e) charger ledit courant aérien de dispositif d'entraînement dans un dispositif de fractionnement d'alkylat ;
(f) récupérer un courant aérien contenant de l'isoparaffine et du HF à partir dudit dispositif de fractionnement d'alkylat ;
(g) recycler ledit courant aérien de l'étape (f) à ladite zone de réaction d'alkylation ; et
(h) récupérer à partir dudit dispositif de fractionnement d'alkylat un courant d'alkylat renfermant de l'essence d'alkylat et une fraction de ladite ASO ayant un point d'ébullition final inférieur à celui de ASO contenue dans ledit courant plus dense de l'étape (b).

2. Un procédé selon la revendication 1 dans lequel ledit fluide d'entraînement de l'étape (c) comprend de l'isobutane ou du n-butane.

3. Un procédé selon la revendication 1, dans lequel ledit fluide d'entraînement de l'étape (c) comprend un produit alkylé formé par réaction d'une isoparaffine avec une oléfine.

4. Un procédé selon l'une quelconque des revendications précédentes, dans lequel ladite d'étape d'entraînement de HF (c) fournit un courant intermédiaire contenant moins de 25 % en poids de HF.

5. Un procédé selon l'une quelconque des revendications précédentes, dans lequel ladite étape d'entraînement de HF (c) fournit un courant intermédiaire contenant moins de 10 % en poids d'acide fluorhydrique.

6. Un procédé selon l'une quelconque des revendications précédentes dans lequel ladite étape d'entraînement de HF (c) fournit un courant intermédiaire contenant moins de 5 % en poids d'acide fluorhydrique.
